# EUROPEAN PATENT SPECIFICATION

(11) **EP 1 841 718 B1**
(45) Date of publication and mention of the grant of the patent: **25.06.2014**
(21) Application number: 05825211.5
(22) Date of filing: 28.12.2005
(51) Int. Cl.: C07C 4/04, C10G 51/02

(54) **PROCESS FOR THE PREPARATION OF LOWER OLEFINS FROM HEAVY WAX**
VERFAHREN ZUR HERSTELLUNG NIEDERER OLEFINE AUS SCHWERWACHS
PROCÉDÉ DE PRODUCTION D'OLÉFINES INFÉRIEURES À PARTIR DE CIRE LOURDE

(30) Priority: 30.12.2004 EP 04107066
(43) Date of publication of application: 10.10.2007
(73) Proprietor: Shell Internationale Research Maatschappij B.V., 2596 HR Den Haag (NL)
(72) Inventor: CRUIJSBERG, Emil, Eduard, Badhuisweg 3, 1031 CM Amsterdam (NL); VAN WESTRENEN, Jeroen, NL-1031 CM Amsterdam (NL)
(74) Representative: Matthezing, Robert Maarten
(86) International application number: PCT/EP2005/057198
(87) International publication number: WO 2006/070006

(56) References cited:
- EP-A- 0 161 705
- EP-A- 0 584 879
- WO-A-99/37737
- GB-A- 686 000
- US-A1- 2002 111 521
- US-A1- 2004 209 964

## Description

The present invention relates to a process for the preparation of lower olefins from a heavy synthetic oil fraction prepared in a Fischer Tropsch process. The process according to the invention comprises mild thermal cracking of the heavy synthetic oil fraction, followed by short residence, high temperature thermal cracking of the product obtained in the first process.

Lower olefins, i.e. olefins having from 2 to 4 carbon atoms, more particularly ethene and/or propene, are very suitable as starting materials in a large number of chemical processes, such as alkylation, oligomerization and polymerisation processes. At the present moment, the main commercial preparation process for these lower olefins is a thermal cracking process in which a hydrocarbon feed, especially ethane or crude oil derived naphtha, is thermally cracked in a short residence, high temperature thermal cracking step. The thermal cracking process, also called pyrolysis process, is a gas phase process and is usually carried out in the presence of an inert gas, often steam or nitrogen, especially steam. In the latter case reference is often made to a steam cracking process. Such a process is commercially applied in a large number of petrochemical complexes.

There is a clear interest in hydrocarbon feedstreams which show a high selectivity towards lower olefins and avoiding as much as possible the formation of methane and/or higher hydrocarbons, especially aromatic hydrocarbons. In addition, the formation of coke should be avoided as much as possible, while a high conversion of the hydrocarbon feedstream should be obtained.

US 2002/0111521 discloses a process for preparing olefins. A Fischer Tropsch waxy product primarily containing C₂₀ to C₅₀ linear paraffins is subjected to catalytic cracking.

WO 99/37737 relates to a process for thermally cracking a Fischer Tropsch derived wax. The Fischer Tropsch derived wax may have a congealing temperature of between 70°C and 110°C. After thermal cracking a lighter wax with a lower congealing temperature is obtained.

US 2004/0209964 discloses a process in which a fraction of a Fischer Tropsch product is subjected to mild thermal cracking. At least 95% by weight of the Fischer Tropsch fraction consists of hydrocarbons containing 15 carbon atoms or more. After hydrogenation and mild thermal cracking a product is separated which comprises C₅+ linear olefins.

GB 686 000 relates to a process in which a paraffin wax is subjected to vapour phase cracking. The material that does not undergo evaporation in the flash evaporator unit is referred to as "evaporator residue". This residue is subjected to a thermal treatment which is also referred to as "soaking" or "thermal soaking". In a next step the soaked residue is subjected to further flash evaporation. The process of GB 686 000 yields long chain olefins, such as olefins containing 15 or more.

In EP 161 705 it has been disclosed that a fraction of the product of a Fischer Tropsch process may be used as a hydrocarbon feed in a thermal cracking process. This reference especially relates to the preparation of C₁₀-₂₀ olefins from the C₂₀₊ hydrocarbon fraction made in the Fischer Tropsch process by mild thermal cracking. The C₁₉₋ fraction may be converted into lower olefins by means of steam cracking. The lower olefins thus prepared can be oligomerized to prepare a mixture of olefins which consists partly of C₁₀-C₂₀ olefins.

In EP 584 879 it has been described that the selectivity of a steam cracking process towards lower olefins, also at high conversion levels, can be further and significantly increased when use is made of a synthetic oil fraction such as a Fischer Tropsch product as a hydrocarbon feed in the steam cracking process, which synthetic oil fraction has been hydro-processed. Suitable hydrogenation processes include hydrogenation, hydroisomerisation and/or hydrocracking. Preferred boiling ranges of the starting material are between 30 and 350°C, more preferably between 30 and 200 °C.

In the known processes in which a Fischer Tropsch fraction is used as a feed for a thermal cracking process to prepare lower olefins, the feed is essentially a non-residual feed, i.e. the feed does not contain any hydrocarbons boiling above 500 °C, or even 600 °C. In the case that certain amounts of very heavy hydrocarbons would be present in the feed, e.g. more than a few wt percent of material boiling above 500 or even 600 °C, such a feed is not very suitable any more for a steam cracking process, as such feed streams would result in large amounts of liquid product during the short residence time high temperature thermal cracking process. This will result in a large amount of unconverted feed. In addition, more coke formation may occur, thus requiring more frequent decoking operations.

It has now been found that in the case in which larger amounts of very heavy hydrocarbons are present in a Fischer Tropsch feed, especially more than 5 wt% based on total feed of material boiling above 550 °C, such a feed, before being processed in a short residence time, high temperature thermal cracking process, is advantageously pre-treated in a mild thermal cracking process. During such a pre-treatment especially the largest hydrocarbon molecules are cracked, resulting in a considerable decrease of the amount of 550°C+ material in the feed. A feedstream thus being treated can be used without further problems in a short residence time, high temperature thermal cracking process, e.g. a steam cracking process, for conversion into lower olefins, i.e. olefins having from 2 to 4 carbon atoms, more particularly ethene and/or propene. In this way a very high yield of lower olefins is obtained, the amount of undesired by-products as methane and/or aromatic compounds, is relatively low and coke formation is also relatively low.

Thus, the present process concerns a process for the preparation of lower olefins from a synthetic oil fraction prepared in a Fischer Tropsch process, the process comprising mild thermal cracking of the synthetic oil fraction, followed by short residence time, high temperature thermal cracking of the product obtained in the mild thermal cracking step, in which process at least 5 wt% of the synthetic oil fraction has a boiling point above 550 °C.

The process of the invention makes it possible to use the complete hydrocarbonaceous product, including the fraction boiling above 550°C, or even boiling above 650 °C, of a Fischer Tropsch process for the production of lower olefins. In the usual commercial processes for making lower olefins from hydrocarbon feedstocks, it is difficult to use material boiling above 550 °C and certainly material boiling above 650 °C. In the present process this high boiling material is converted into lower boiling material, thus making it better suitable as steamcracker feed. Lower olefins can be made in a very high yield due to a high conversion of the feed in combination with a high selectivity for lower olefins. In addition, coke formation is low in comparison with crude oil based naphtha fractions. Methane formation as well as the formation of higher hydrocarbons, especially aromatic hydrocarbons is relative low.

The overall conversion of the starting synthetic oil fraction into lower olefins, i.e. C₂-C₄ olefins, varies between 40 and 80% based on the weight of the starting material and the weight of the lower olefins produced, usually between 55 and 70%.

The process of the present invention is especially suitable for feedstocks in which the part of the synthetic oil fraction having a boiling point above 550 °C is at least 10 wt% of the total synthetic oil fraction, preferably 30 wt%, more preferably at least 50 wt%.

In one embodiment of the invention the complete Fischer Tropsch product is sent to the mild thermal cracking step. In a preferred embodiment only the C₅₊ fraction is sent to the mild thermal cracking step. This separation can be done easily by cooling down the product of the Fischer Tropsch reaction and separating the gaseous fraction from the liquid fraction. By choosing the temperature and the pressure of the reaction product correctly, a separation can be made between unconverted syngas, inerts and C₁ to C₄ hydrocarbons and the C₅₊ fraction. Such a separation has the additional advantage that also Fischer Tropsch product water will be removed from the feed product. By choosing the temperature and/or the pressure differently, it is also possible to isolate the e.g. C₃₊ fraction or the C₁₀₊ or the C₂₀₊ fraction. In a further preferred process only the more heavy products from the Fischer Tropsch process are sent to the mild thermal cracking process, e.g. only the 250 °C fraction, or more preferably the 350 °C fraction.

The present process is especially suitable for Fischer Tropsch reaction products which are made under such circumstances that an extremely heavy product is obtained. In order to minimise the amount of C₁-C₄ product which is formed in the Fischer Tropsch process, conditions and catalyst are used nowadays in which substantial parts of the Fischer Tropsch products exists of material boiling above 650 °C. Thus, in a preferred embodiment the starting synthetic oil fraction has a boiling point above 650 °C, the amount boiling above 650 °C suitably being at least 10 wt% of the total synthetic oil fraction, preferably at least 20 wt%, more preferably at least 40 wt%, still more preferably at least 60 wt%. In a further preferred embodiment the starting synthetic oil fraction comprises at least 10 wt% of material boiling above 750 °C based on the total synthetic oil fraction, preferably at least 40 wt%, more preferably at least 70 wt%.

The product of the Fischer Tropsch reaction mainly consists of paraffin, olefins and oxygenates. These compounds in general have the same carbon chain structure, the difference being the functionality of the atoms attached to the carbon chain. Under exceptional circumstances naphthenic and/or aromatic compounds may be formed (especially when relatively high temperatures are used), however, these compounds are not desired. It is preferred that the Fischer Tropsch compounds are straight chain compounds, or straight chain compounds having up to three, preferably up to two, more preferably up till one branch, preferably methyl or ethyl branches, especially methyl branches. Straight chain, i.e. normal paraffins, are preferred. Thus, preferably the synthetic oil fraction to be used in the claimed process, after hydrogenation, comprises at least 90 wt% of paraffins, preferably 95 wt%. The amount of naphthenic and aromatic compounds together is preferably at most 5 wt%, more preferably at most 1 wt%. The amount of normal compounds (paraffins, olefins and oxygenates) is suitably as high as possible. Thus, the synthetic oil fraction comprises at least 50 wt% normal compounds, preferably at least 65 wt%, more preferably at least 80 wt%. The amount of olefins in the synthetic oil fraction is suitably less than 30 wt% of total fraction, preferably less than 20 wt%, more preferably less than 10 wt%. The amount of oxygenates is suitably less than 15 wt% based on total fraction, preferably less than 7.5 wt%, more preferably less than 5 wt%.

In a further preferred embodiment the synthetic oil fraction is hydroprocessed before the mild thermal cracking process, preferably hydrogenated. This results in a further increase of the yield of ethene/propene. Hydrogenation is suitable being carried out at elevated temperature and pressure in the presence of hydrogen and a hydrogenation catalyst. The action of the hydrogenation stage is, for example, to hydrogenate any unsaturated hydrocarbons and oxygenates present in the synthetic oil without substantial hydroisomerisation and/or hydrocracking occurring. Preferably, the hydrogenation is carried out at a temperature of from 100 °C to 300 °C, more preferably at a temperature of from 150 °C to 275 °C, in particular of from 175 °C to 250 °C. The hydrogenation may be carried out at a relatively wide range of pressures, but preferably, the hydrogenation is carried out at a hydrogen partial pressure of from 5 bar to 150 bar, more preferably of from 20 bar to 120 bar.

The hydrogenation may be carried out using any type of catalyst bed arrangement, such as a fluidized bed, moving bed, slurry phase bed or a fixed bed, each type of catalyst bed having its own characteristic advantages and disadvantages. However, preferably a fixed catalyst bed is applied. It is to be understood that the reaction conditions, such as temperature, pressure and space velocity, may vary according to the specific type of catalyst bed being used. If a fixed catalyst bed is being used, the synthetic oil feed is preferably provided at a weight hourly space velocity of from 0.1 kg/l/h to 5 kg/l/h, more preferably at a weight hourly space velocity of from 0.25 kg/l/h to 2.5 kg/l/h. Hydrogen may be applied to the hydrogenation stage at a gas hourly space velocity in the range of from 100 to 10000 Nl/l/hr, more preferably from 250 to 5000 Nl/l/hr. The ratio of hydrogen to the feed may range from 100 to 5000 Nl/kg and is preferably from 250 to 2500 Nl/kg.

Hydrogenation catalysts are well known in the art and are commercially available in a large variety of compositions. Typically, the hydrogenation catalyst comprises as catalytically active component one or more metals selected from Groups VIb and VIII of the Periodic Table of the Elements, in particular one or more metals selected from molybdenum, tungsten, cobalt, nickel, ruthenium, iridium, osmium, platinum and palladium. Preferably, the catalyst comprises one or more metals selected from nickel, platinum and palladium as the catalytically active component. A particularly suitable catalyst comprises nickel as a catalytically active component.

Hydrogenation catalysts typically comprise a refractory metal oxide or silicate as a carrier. Suitable carrier materials include silica, alumina, silica-alumina, zirconia, titania and mixtures thereof. Preferred carrier materials for inclusion in the hydrogenation catalyst are silica, alumina and silica-alumina.

The hydrogenation catalyst may comprise the catalytically active component in an amount of from 0.05 to 70 parts by weight, preferably from 0.1 to 50 parts by weight, calculated as metal(s) per 100 parts by weight of total catalyst. The amount of catalytically active metal present in the catalyst will vary according to the specific metal concerned. A particularly suitable hydrogenation catalyst comprises nickel in an amount in the range of from 30 to 70 parts by weight, calculated as metal per 100 parts by weight of total catalyst.

Suitable hydrogenation catalysts are available commercially, or may be prepared by methods well known in the art, for example comulling, impregnation or precipitation.

In the case that more severe conditions are used for the hydrogenation reaction, e.g. temperatures between 275 and 400 °C, especially in combination with acidic carriers as amorphous silica/alumina and especially zeolites, also hydroisomerisation will occur. In general the amount of hydroisomerisation will be kept less than 40 wt% based on feed, preferably less than 10 wt%, more preferably less than 5 wt%. In all cases, hydrocracking is to be kept at a relatively low level. Thus, the conversion of any material boiling above 360 °C into material boiling below 360 °C is suitably less than 15 wt%, preferably less than 10 wt%, more preferably less than 3 wt%.

In another embodiment oxygenates may be removed by a dehydration process. This process is suitably carried out in the presence of a dehydration catalyst. Suitable dehydration catalysts are well known in the art. Especially acid catalysts are used. Removal of oxygenates results in a higher yield of ethene/propene, while the oxygen content of the ethene/propene fraction will be deceased. This is important as in certain catalytic polymerization processes the catalyst may very very sensitive to even trace amounts of oxygen.

The initial boiling point of the synthetic oil to be used in the process according to the invention is suitably at least 30 °C. In a preferred embodiment the initial boiling point is at least 250 °C, more especially at least 350 °C, more preferably at least 450 °C, even more preferably at least 550 °C. By removing a part of the low boiling fraction, a smaller thermal cracking unit may be used. The untreated initial feed, especially the feed boiling between 30 and 350, preferably 450, more preferably 550°C, may be combined with the thermally cracked fraction, and the combined stream can be used as feed for the short residence time/high temperature thermal cracking process.

The mild thermal cracking process to be used in the process according to the present invention may be any mild thermal cracking process known in the art. Very suitably it is done by a furnace cracking process, but it is preferably a soaker visbreaking process. In the soaker visbreaking process the feed is heated in a furnace to a temperature suitably between 380 and 500 °C, preferably between 400 and 480 °C, suitably using a residence time of up till 5 minutes, preferably up till 3 minutes, followed by further conversion in a soaker vessel. The residence time in the soaker vessel is suitably between 0.5 and 2 hours. The pressure is usually between 3 and 10 bar. The conversion (of material boiling above 550 °C) obtained is suitably at least 20 wt%, preferably at least 60 wt%. Especially the conversion is between 30 and 98 wt% of the material boiling above 550 °C, preferably between 60 and 95 wt%. Preferably at least 99 wt% of the material boiling above 750 °C is removed, more preferably at least 99 wt% of the material boiling above 650 °C is removed. In the case of furnace cracking the temperature is suitably between 420 and 540 °C, preferably between 460 and 520 °C, the pressure is suitably between 5 and 50 bar, preferably between 15 and 20 bar and the residence time is suitably between 1 and 15 minutes, especially between 4 and 12 minutes. The conversion levels are the same as for the soaker process.

The product obtained in the mild thermal cracking process may be used directly in the short residence, high temperature cracking process, but is preferably separated into a light fraction and a heavy fraction. The separation can be done by means of any equipment known in the art to separate feedstreams into fractions having different boiling ranges, e.g. distillation equipment, but is preferably done by means of a flash separation. The light fraction suitably boils up till 450 °C, preferably up till 500 °, more preferably up till 550 °C or even 650 °C. The heavy fraction may be recycled to the mild thermal cracking step. In the case of a recycle it is preferred to remove between 5 and 40 wt% of the stream as a bleed stream. Such a bleed stream is advantageously used as fuel, either in the mild thermal cracking step or in the second cracking step.

In a further embodiment of the invention, the product obtained in the mild thermal cracking process may be hydrogenated before introduction into the short residence, high temperature thermal cracking process. This can be done in exactly the same way as described above (including all preferred ranges) for the hydrogenation of the starting Fischer Tropsch product. In this way the olefins formed during the mild thermal cracking step are converted into saturated paraffins, resulting in a further improvement (selectivity to lower olefins, reduced coke formation) of the second cracking step.

The production of lower olefins, in particular ethene and propene, is in general achieved by pyrolyzing the Fischer-Tropsch derived hydrocarbons.

Pyrolysis comprises steam cracking, which is thermal cracking of hydrocarbons in the presence of steam and if desired a dilution gas. The process comprises a convection zone, a cracking zone, a cooling zone and a separation zone. The pyrolysis furnace comprises the convection zone and the cracking zone. The convection zone usually comprises a first preheating zone and a second preheating zone. Generally, feed is heated in the first preheating zone, and dilution gas is added to the feed before the (liquid and gas) mixture of feed and dilution gas is sent to the second preheating zone.

Furnaces designed for treating gasoil and even heavier feed streams will have a larger heat transfer surface area in the first preheating zone than furnaces designed for a light feed, e.g. naphtha, as the main aim of the first preheating zone is vaporizing the feed and heating the feed.

A furnace designed for treating gaseous feed, will have a smaller heat transfer surface area in the first preheating zone than a furnace designed for liquid feed as a gaseous feed does not need to be vaporized. It is to be understood that the scope of the steam cracking process may include any number and types of process steps between each described process step or between a described source and destination within a process step.

Usually and preferably, all product of a process step will be subjected to the next process step. However, it is possible to send only part of the product of a process step to the next process step.

Feed can be introduced into the process at further inlets besides the standard inlet and the inlet where feed is introduced together with steam and/or dilution gas. However, it is preferred to introduce feed only at the standard inlet of the convection zone and further feed together with steam and/or dilution gas.

Dilution gas (usually steam) can be added at a single inlet, or can be added via several inlets. However, it is preferred to add dilution gas at a single inlet.

The convection zone generally comprises a first preheating zone and a second preheating zone between which is located an inlet for steam and optionally dilution gas. In the first preheating zone, the feed is heated. After the first preheating zone, steam and optionally dilution gas is added to the feed and the mixture obtained can be heated further in the second preheating zone to a temperature just below the temperature at which cracking starts to occur. The temperature of the product obtained from the convection zone will usually be of from 400 to 800 °C, depending upon the feed, more specifically of from 450 to 750 °C.

The pyrolysis furnace may be any type of conventional olefins pyrolysis furnace designed for pyrolizing heavy feed and operated for production of lower boiling products such as olefins, especially including a tubular steam cracking furnace. The tubes within the convection zone of the pyrolysis furnace may be arranged as a bank of tubes in parallel, or the tubes may be arranged for a single pass of the feedstock through the convection zone. Within each bank, the tubes may arranged in a coil or serpentine type arrangement. At the inlet, the feed may be split among several tubes, or may be fed to one single pass tube through which all the feed flows from the inlet to the outlet of the first stage preheater. Preferably, the first and/or second preheating zone of the convection zone comprise a multiple pass tubular reactor in which feed is passed through the first and/or the second preheating zone via more than one tube. Multiple pass tubular reactors often contain tubes having connections at their ends leading feed from the one tube to the next tube until the feed is sufficiently heated to be mixed with dilution gas and be passed to the second preheating zone, or to be sent to the cracking zone.

The pressure and temperature at which the feed is fed to the inlet of the first preheating zone is not critical, typically the temperature will be of from 0 to 300 °C.

The optimal temperature to which the feed is heated in the first preheating zone will depend upon the pressure of the feed, and the performance and operation of the remainder of the process. The product of the first preheating zone will generally have an exit temperature of at least 150 °C such as 195 °C. The upper range on the temperature of the feed in the first preheating zone is limited to the point at which the stability of the feed is impaired. At a certain temperature, the coking propensity of the feed increases. This temperature limit would apply to both the first and the second preheating zone and all tubes in these zones. Preferably, the exit temperature of the feed within the first preheating zone is not more than 520 °C, preferably not more than 500 °C, more preferably not more than 450 °C or even 400 °C.

The heating elements in the first and second preheating zone in the convection zone is typically a bank of tubes, wherein the contents in the tubes are heated primarily by convective heat transfer from the combustion gas exiting from the cracking zone of the pyrolysis furnace, so-called flue gas. However, different heating elements can be used as well.

The pressure within the first and second preheating zone is not particularly limited. The pressure is generally within a range of from 4 to 21 bar, more preferably of from 5 to 13 bar.

In the process of the present invention part of the heavy hydrocarbons obtained by Fischer-Tropsch synthesis as the feed is introduced via the standard feed inlet of the convection zone, and if desired part of the feed is introduced further downstream in the convection zone.

Steam gas is added to the convection zone. This can be done preferably in or before the second preheating zone of the convection zone. Other dilution gas is preferably added at a point external to the pyrolysis furnace for ease of maintaining and replacing equipment.

The dilution gas is a vapour at the injection point into the convection zone. Examples of dilution gases are methane, ethane, nitrogen, hydrogen, natural gas, dry gas, refinery off gases, and a vaporized naphtha. Preferably, the steam is superheated steam.

Typical dilution gas temperatures at the dilution gas/feed junction range of from 140 °C to 800 °C, more preferably of from 150 °C to 780 °C, more preferably of from 200 to 750 °C.

The pressure of dilution gas is not particularly limited, but is preferably sufficient to allow injection. Typical dilution gas pressures added to the crude oil is generally within the range of from 6 to 15 bar.

It is desirable to add steam and optionally dilution gas between the first preheating zone and the second preheating zone in an amount which will generally be not more than 1 kg of dilution gas per kg of feed. However, there can be circumstances in which a higher amount of dilution gas can be advantageous.

The mixture of dilution gas and feed is fed to the second preheating zone where the mixture is heated further. The mixture generally comprises not more than 50 wt% liquid Fischer-Tropsch hydrocarbons. Preferably not more than 25 wt%, most preferably not more than 10 wt% Tubes of the second preheating zone can be heated by the flue gases from the cracking zone of the furnace. In the second preheating zone (super heater), the mix is fully preheated to near or just below a temperature at which substantial feedstock cracking and associated coke laydown in the preheater would occur such as 450 to 550 °C, preferably 460-500 °C, such as 490 °C.

Subsequently, the product of the convection zone is sent to the cracking zone. The temperature of the mixture of steam and feed is increased further under controlled residence time, temperature profile and partial pressure. The exit temperature of the product obtained in the cracking zone is generally of from 700 to up to 1000 °C. more specifically of from 750 to 950 °C. The pressure is generally within a range of from 2 to 25 bar, more preferably of from 3 to 18 bar.

The reactions in the cracking zone are highly endothermic, and therefore a high rate of energy input is needed.

On leaving the cracking zone, the products are generally immediately cooled. The temperature of the product will usually be reduced to a temperature of from 200 to 700 °C, more specifically of from 250 to 650 °C to prevent degradation by secondary reactions. Cooling of the product obtained in the cracking zone can be done in any way suitable, such as by direct quenching or indirect quenching.

The cooled product is subsequently separated into the desired end-products. Separation of the desired end-products can start at cooling where heavy components can be removed. Further, during cooling the gas obtained can be compressed, and acids and water can be removed. Subsequently, the product can be dried and uncracked feed, ethane and propane may be recovered for recycling as pyrolysis feed. The cracking severity affects the composition of the product obtained.

Products of an olefins pyrolysis furnace include, but are not limited to, ethene, propene, butadiene, benzene, hydrogen, and methane, and other associated olefinic, paraffinic, and aromatic products. Ethene generally is the predominant product, typically ranging from 15 to 60 %wt, based on the weight of the feed.

In a typical work-up, the product of the cracking zone is cooled with the help of a water quench, followed by multi-stage compression typically in 4 to 6 stages. Before the last compressor stage, the gas is treated with caustic to remove hydrogen sulphide and carbon dioxide. Acetylenes may be hydrogenated with hydrogen-rich compressor gas. After the last compression stage, the cracked gas is typically dehydrated by chilling and dried by use of molecular sieves. Methane and hydrogen can be removed in a demethanizer. In a demethanizer, the hydrocarbons containing 2 carbon atoms are produced overhead and the hydrocarbons containing 3 carbon atoms or more is a bottom product. The overhead stream can be hydrogenated to remove acetylene and then fractionated to produce ethene and ethane. The ethane can be recycled. The bottom product can be further fractionated, if appropriate, to remove heavy ends including compounds containing 4 carbon atoms or more. The overhead stream from a depropanizer can be hydrogenated to remove methylacetylene and propadiene, which can be recovered for sale or removed via other means. Propene can be obtained as overhead stream from the depropanizer, and the bottom propane fraction can be recycled.

It is a preferred characteristic of the Fischer-Tropsch hydrocarbons that they are essentially free of aromatic compounds, nitrogen comprising compounds and sulphur comprising compounds.

The Fischer-Tropsch hydrocarbons to be used according to the invention as a feed for steam for the production of lower olefins, are produced in a Fischer-Tropsch synthesis. Fischer-Tropsch synthesis of hydrocarbons is a well known process. In the Fischer-Tropsch synthesis the starting material is a hydrocarbonaceous feed.

The hydrocarbonaceous feed suitably is methane, natural gas, associated gas or a mixture of C₁-₄ hydrocarbons. The feed comprises mainly, i.e. more than 90 v/v%, especially more than 94%, C₁-₄ hydrocarbons, especially comprises at least 60 v/v percent methane, preferably at least 75 percent, more preferably 90 percent. Very suitably natural gas or associated gas is used. Suitably, any sulphur in the feedstock is removed.

The partial oxidation of this hydrocarbons feed, producing mixtures of especially carbon monoxide and hydrogen, can take place according to various established processes. These processes include the Shell Gasification Process. A comprehensive survey of this process can be found in the Oil and Gas Journal, September 6, 1971, pp 86-90.

The oxygen containing gas is air (containing about 21 vol. percent of oxygen), oxygen enriched air, suitably containing up to 70 percent, or substantially pure air, containing typically at least 95 vol.% oxygen. Oxygen or oxygen enriched air may be produced via cryogenic techniques, but could also be produced by a membrane based process, e.g. the process as described in WO 93/06041. To adjust the H₂/CO ratio in the syngas, carbon dioxide and/or steam may be introduced into the partial oxidation process. Preferably up to 15% volume based on the amount of syngas, preferably up to 8% volume, more preferable up to 4% volume, of either carbon dioxide or steam is added to the feed. Water produced in the hydrocarbon synthesis may be used to generate the steam. As a suitable carbon dioxide source, carbon dioxide from the effluent gasses of the expanding/combustion step may be used. The H₂/CO ratio of the syngas is suitably between 1.3 and 2.1, preferably between 1.4 and 2.0. If desired, (small) additional amounts of hydrogen may be made by steam methane reforming, preferably in combination with the water shift reaction. Any carbon monoxide and carbon dioxide produced together with the hydrogen may be used in the hydrocarbon synthesis reaction or recycled to increase the carbon efficiency. Additional hydrogen manufacture may be an option.

The percentage of light hydrocarbonaceous feed which is converted in the first step of the process of the invention is suitably 50-99% by weight and preferably 80-98% by weight, more preferably 85-96% by weight.

The gaseous mixture, comprising predominantly hydrogen, carbon monoxide and optionally nitrogen, is contacted with a suitable catalyst in the catalytic conversion stage, in which the hydrocarbons are formed. Suitably at least 70 v/v% of the syngas is contacted with the catalyst, preferably at least 80%, more preferably at least 90, still more preferably all the syngas.

The catalysts used in for the catalytic conversion of the mixture comprising hydrogen and carbon monoxide are known in the art and are usually referred to as Fischer-Tropsch catalysts. Catalysts for use in the Fischer-Tropsch hydrocarbon synthesis process frequently comprise, as the catalytically active component, a metal from Group VIII of the Periodic Table of Elements. Particular catalytically active metals include ruthenium, iron, cobalt and nickel. Cobalt is a preferred catalytically active metal.

The catalytically active metal is preferably supported on a porous carrier. The porous carrier may be selected from any of the suitable refractory metal oxides or silicates or combinations thereof known in the art. Particular examples of preferred porous carriers include silica, alumina, titania, zirconia, ceria, gallia and mixtures thereof, especially silica and titania.

The amount of catalytically active metal on the carrier is preferably in the range of from 3 to 300 pbw per 100 pbw of carrier material, more preferably from 10 to 80 pbw, especially from 20 to 60 pbw.

If desired, the catalyst may also comprise one or more metals or metal oxides as promoters. Suitable metal oxide promoters may be selected from Groups IIA, IIIB, IVB, VB and VIB of the Periodic Table of Elements, or the actinides and lanthanides. In particular, oxides of magnesium, calcium, strontium, barium, scandium, yttrium. lanthanum, cerium, titanium, zirconium, hafnium, thorium, uranium, vanadium, chromium and manganese are most suitable promoters. Particularly preferred metal oxide promoters for the catalyst used to prepare the waxes for use in the present invention are manganese and zirconium oxide. Suitable metal promoters may be selected from Groups VIIB or VIII of the Periodic Table. Rhenium and Group VIII noble metals are particularly suitable, with platinum and palladium being especially preferred. The amount of promoter present in the catalyst is suitably in the range of from 0.01 to 100 pbw, preferably 0.1 to 40, more preferably 1 to 20 pbw, per 100 pbw of carrier.

The catalytically active metal and the promoter, if present, may be deposited on the carrier material by any suitable treatment, such as impregnation, kneading and extrusion. After deposition of the metal and, if appropriate, the promoter on the carrier material, the loaded carrier is typically subjected to calcination at a temperature of generally from 350 to 750 °C, preferably a temperature in the range of from 450 to 550 °C. The effect of the calcination treatment is to remove crystal water, to decompose volatile decomposition products and to convert organic and inorganic compounds to their respective oxides. After calcination, the resulting catalyst may be activated by contacting the catalyst with hydrogen or a hydrogen-containing gas, typically at temperatures of about 200 to 350 °C.

The catalytic conversion process may be performed under conventional synthesis conditions known in the art. Typically, the catalytic conversion may be effected at a temperature in the range of from 100 to 600 °C, preferably from 150 to 300 °C, more preferably from 180 to 280 °C. Typical total pressures for the catalytic conversion process are in the range of from 1 to 200 bar absolute, more preferably from 10 to 70 bar absolute. In the catalytic conversion process mainly (at least 70 wt%, preferably 85 wt% of C₅₊ hydrocarbons are formed.

Preferably, a Fischer-Tropsch catalyst is used, which yields substantial quantities of normal (and also iso-) paraffins, more preferably substantially normal paraffins. A most suitable catalyst for this purpose is a cobalt-containing Fischer-Tropsch catalyst. The Fischer-Tropsch hydrocarbons comprise generally C₄-C₂₀₀, preferably C₄-C₁₀₀ hydrocarbons. Normally liquid Fischer-Tropsch hydrocarbons are suitably C₄-₂₅ hydrocarbons, especially C₅-₂₀ hydrocarbons. These hydrocarbons are liquid at temperatures between 5 and 30 °C (1 bar), especially at about 20 °C (1 bar), and usually are paraffinic of nature, while up to 24 wt%, preferably up to 12 wt%, of either olefins or oxygenated compounds may be present. Depending on the catalyst and the process conditions used in the Fischer Tropsch reaction, normally gaseous hydrocarbons, normally liquid hydrocarbons and optionally normally solid hydrocarbons are obtained.

## Claims

1. Process for the preparation of lower olefins from a synthetic oil fraction prepared in a Fischer Tropsch process, the process comprising mild thermal cracking of the synthetic oil fraction, followed by short residence time, high temperature thermal cracking of the product obtained in the mild thermal cracking step, in which process at least 5 wt% of the synthetic oil fraction has a boiling point above 550°C.

2. Process according to claim 1, in which the part of the synthetic oil fraction having a boiling point above 550 °C is at least 10 wt% of the total synthetic oil fraction, preferably at least 30 wt%, more preferably at least 50 wt%.

3. Process according to claim 1 or 2, in which the synthetic oil fraction has a boiling point above 650 °C, the amount boiling above 650 °C preferably being at least 20 wt% of the total synthetic oil fraction, more preferably at least 40 wt%, still more preferably at least 60 wt%.

4. Process according to any of claims 1 to 3, in which the Fischer Tropsch process comprises reaction of carbon monoxide and hydrogen over a cobalt or iron based Fischer Tropsch catalyst, preferably a cobalt based catalyst, at a temperature between 150 and 300 °C and a pressure between 5 and 100 bara, preferably between 180 and 280 °C and 20 to 70 bara, preferably a process in which the SFA alpha value based on the C₂₀ and C₃₉ paraffin fractions made in the Fischer Tropsch process is at least 0.90, preferably at least 0.92, more preferably at least 0.94.

5. Process according to any of claims 1 to 4, in which the synthetic oil fraction would comprise at least 90 wt% paraffins after hydrogenation, preferably 95 wt% or in which the synthetic oil fraction comprises at least 50 wt% normal compounds, preferably at least 60 wt%, more preferably at least 70 wt%.

6. Process according to any of claims 1 to 5, in which at least part of the oxygenates and/or olefins, preferably at least a part of the oxygenates, more preferably all oxygenates, are removed in a pre-treatment process, preferably a dehydration process or a hydrotreatment process.

7. Process according to any of claims 1 to 6, in which the initial boiling point of the synthetic oil fraction is at least 250 °C, preferably at least 350 °C, more preferably at least 450 °C.

8. Process according to any of claims 1 to 7, in which the mild thermal cracking process comprises furnace cracking or soaker cracking, preferably in which the furnace cracking is carried out at a temperature between 500 °C and 700 °C and a residence time up till 6 minutes or in which the soaker cracking is done at a temperature between 400 °C and 500 °C and a residence time between 10 and 60 minutes.

9. Process according to claim 8, in which the conversion of 550 °C+ material into 550 °C- material is at least 40%, preferably at least 70%.

10. Process according to any of claims 1 to 9, in which any 550 °C+ material present in the product after mild thermal cracking is separated from the reaction product and recycle to the mild thermal carking step, preferably all 650 °C+ material is recycled.

11. Process according to any of claims 1 to 10, in which the C₂ and C₃ is also send to the high temperature thermal cracking unit or in which liquid hydrocarbons other than the synthetic oil fraction used for the process according to the invention is also sent to the high temperature thermal cracking unit.

12. Process according to any of claims 1 to 11, in which the methane produced in the Fischer Tropsch reaction and any unconverted hydrogen and carbon monoxide is used as fuel for mild thermal cracking reaction and/or the high temperature thermal cracking.

13. Process according to any of claims 1 to 12, in which natural gas condensates and/or ethane/propane extracted from natural gas sources are also send to the high temperature cracking process.

## Patentansprüche

1. Verfahren zur Herstellung niederer Olefine aus einer Syntheseölfraktion, hergestellt in einem Fischer-Tropsch-Verfahren, wobei das Verfahren das milde thermische Cracken der Syntheseölfraktion, gefolgt von einem thermischen Cracken unter hoher Temperatur und kurzer Verweildauer des im Schritt des milden thermischen Crackens erhaltenen Produkts umfasst, in welchem Verfahren mindestens 5 Gew.-% der Syntheseölfraktion einen Siedepunkt über 550 °C besitzen.

2. Verfahren nach Anspruch 1, wobei der Teil der Syntheseölfraktion mit einem Siedepunkt über 550 °C mindestens 10 Gew.-% der gesamten Syntheseölfraktion, vorzugsweise mindestens 30 Gew.-%, stärker bevorzugt mindestens 50 Gew.-% darstellt.

3. Verfahren nach Anspruch 1 oder 2, wobei die Syntheseölfraktion einen Siedepunkt über 650 °C besitzt, wobei die Menge, welche über 650 °C siedet, vorzugsweise mindestens 20 Gew.-% der gesamten Syntheseölfraktion, stärker bevorzugt mindestens 40 Gew.-%, noch stärker bevorzugt mindestens 60 Gew.-% darstellt.

4. Verfahren nach einem der Ansprüche 1 bis 3, wobei das Fischer-Tropsch-Verfahren die Umsetzung von Kohlenmonoxid und Wasserstoff über einem auf Kobalt oder Eisen basierenden Fischer-Tropsch-Katalysator, vorzugsweise einem auf Kobalt basierenden Katalysator, bei einer Temperatur von 150 bis 300 °C und einem Druck von 5 bis 100 bara, vorzugsweise von 180 bis 280 °C und 20 bis 70 bara, umfasst, vorzugsweise ein Verfahren, wobei der SFA-alpha-Wert, basierend auf den C₂₀ und C₃₉-Paraffinfraktionen, welche im Fischer-Tropsch-Verfahren hergestellt werden, mindestens 0,90, vorzugsweise mindestens 0,92, stärker bevorzugt mindestens 0,94 beträgt.

5. Verfahren nach einem der Ansprüche 1 bis 4, wobei die Syntheseölfraktion mindestens 90 Gew.-% Paraffine nach der Hydrierung, vorzugsweise 95 Gew.-%, umfassen würde oder wobei die Syntheseölfraktion mindestens 50 Gew.-% normale Verbindungen, vorzugsweise mindestens 60 Gew.-%, stärker bevorzugt mindestens 70 Gew.-% umfasst.

6. Verfahren nach einem der Ansprüche 1 bis 5, wobei mindestens ein Teil der Oxygenate und/oder der Olefine, vorzugsweise mindestens ein Teil der Oxygenate, stärker bevorzugt die gesamten Oxygenate, in einem Vorbehandlungsverfahren, vorzugsweise einem Dehydratisierungsverfahren oder einem Hydrobehandlungsverfahren, entfernt werden.

7. Verfahren nach einem der Ansprüche 1 bis 6, wobei der Anfangssiedepunkt der Syntheseölfraktion mindestens 250 °C, vorzugsweise mindestens 350 °C, stärker bevorzugt mindestens 450 °C beträgt.

8. Verfahren nach einem der Ansprüche 1 bis 7, wobei das milde thermische Crackverfahren ein Ofen-Cracken oder ein "Soaker-Cracking" umfasst, wobei das Ofen-Cracken vorzugsweise bei einer Temperatur von 500 °C bis 700 °C und einer Verweildauer bis zu 6 Minuten durchgeführt wird, oder wobei das "Soaker-Cracking" bei einer Temperatur von 400 °C bis 500 °C und einer Verweildauer von 10 bis 60 Minuten durchgeführt wird.

9. Verfahren nach Anspruch 8, wobei die Umwandlung von 550 °C+ Material in 550 °C- Material mindestens 40%, vorzugsweise mindestens 70% beträgt.

10. Verfahren nach einem der Ansprüche 1 bis 9, wobei jedwedes 550 °C+ Material, welches im Produkt nach dem milden thermischen Cracken vorhanden ist, vom Reaktionsprodukt abgetrennt und in den Schritt des milden thermischen Crackens rezykliert wird, wobei vorzugsweise das gesamte 650 °C+ Material rezykliert wird.

11. Verfahren nach einem der Ansprüche 1 bis 10, wobei die C2 und C3 ebenfalls zur Einheit für das thermische Cracken unter hoher Temperatur geleitet werden oder wobei die flüssigen Kohlenwasserstoffe, welche nicht die Syntheseölfraktion sind, die für das Verfahren gemäß der Erfindung verwendet wird, ebenfalls zur Einheit für das thermische Cracken unter hoher Temperatur geleitet werden.

12. Verfahren nach einem der Ansprüche 1 bis 11, wobei das in der Fischer-Tropsch-Reaktion hergestellte Methan und jedweder nicht umgesetzter Kohlenwasserstoff und jedwedes nicht umgesetztes Kohlenmonoxid als Brennstoff für die milde thermische Crackreaktion verwendet und/oder für das thermische Cracken unter hoher Temperatur verwendet werden.

13. Verfahren nach einem der Ansprüche 1 bis 12, wobei Erdgaskondensate und/oder Ethan/Propan, welche aus Erdgasquellen gewonnen werden, ebenfalls zum Verfahren des Crackens unter hoher Temperatur geleitet werden.

## Revendications

1. Procédé pour la préparation d'oléfines à bas poids moléculaire à partir d'une fraction en huile synthétique selon le procédé de Fischer Tropsch, le procédé comprenant un craquage thermique doux de la fraction en huile synthétique, suivi d'un temps de séjour de court, un craquage thermique à haute température du produit issu de l'étape de craquage thermique doux, procédé dans lequel au moins 5% en poids de la fraction en huile synthétique présente un point d'ébullition au-dessus de 550°C.

2. Procédé selon la revendication 1, dans lequel la fraction en huile synthétique présentant un point d'ébullition au-dessus de 550°C correspond à au moins 10 % en poids de la fraction totale en huile synthétique, de préférence 30% en poids, de manière plus préférentielle au moins 50% en poids.

3. Procédé selon la revendication 1 ou 2, dans lequel la fraction en huile synthétique présente un point d'ébullition au-dessus de 650°C, la quantité bouillant à 650°C représentant de préférence au moins 20% en poids de la fraction totale en huile synthétique, de manière plus préférentielle au moins 40% en poids, de manière encore plus préférentielle au moins 60% en poids.

4. Procédé selon l'une quelconque des revendications 1 à 3, dans lequel le procédé de Fischer Tropsch comprend la réaction du monoxyde de carbone avec de l'hydrogène en présence d'un catalyseur Fisher Tropsch à base de fer ou de cobalt, de préférence un catalyseur à base de cobalt, à une température comprise entre 150 et 300°C et une pression comprise entre 5 et 100 bars, de préférence entre 180 et 280°C et entre 20 et 70 bars, procédé dans lequel de préférence la valeur alpha SFA sur base des fractions en paraffines C₂₀ et C₃₉ produites par le procédé de Fisher Tropsch est d'au moins 0,90, de préférence d'au moins 0,92, de manière plus préférentielle d'au moins 0,94.

5. Procédé selon l'une quelconque des revendications 1 à 4, dans lequel la fraction en huile synthétique doit comprendre au moins 90% en poids de paraffines après hydrogénation, de préférence 95% en poids, ou dans lequel la fraction en huile synthétique comprend au moins 50% en poids de composés normaux, de préférence au moins 60% en poids, de manière plus préférentielle au moins 70% en poids.

6. Procédé selon l'une quelconque des revendications 1 à 5, dans lequel au moins une partie des composés oxygénés et/ou des oléfines, de préférence au moins une partie des composés oxygénés, de manière plus préférentielle tous les composés oxygénés, sont éliminés par un procédé de prétraitement, de préférence un procédé de déshydratation ou un procédé d'hydrotraitement.

7. Procédé selon l'une quelconque des revendications 1 à 6, dans lequel le point d'ébullition initial de la fraction en huile synthétique est d'au moins 250°C, de préférence d'au moins 350°C, de manière plus préférentielle d'au moins 450°C.

8. Procédé selon l'une quelconque des revendications 1 à 7, dans lequel le procédé de craquage thermique doux comprend une étape de craquage par four ou de craquage par maturateur de préférence dans lequel le craquage par four est mis en oeuvre à une température comprise entre 500 et 700°C et un temps de séjour jusqu'à 6 minutes ou dans lequel le craquage par maturateur est réalisé à une température comprise entre 400 et 500°C et un temps de séjour entre 10 et 60 minutes.

9. Procédé selon la revendication 8, dans lequel la conversion d'un matériau à 550°C+ en un matériau à 550°C- est d'au moins 40%, de préférence d'au moins 70%.

10. Procédé selon l'une quelconque des revendications 1 à 9, dans lequel tous les matériaux à 550°C+ présents dans le produit après craquage thermique doux est séparé du produit de réaction et se recycle vers l'étape de craquage thermique doux, de préférence tous les matériaux à 650°C+ sont recyclés.

11. Procédé selon l'une quelconque des revendications 1 à 10, dans lequel les C₂ et C₃ sont aussi envoyés vers l'unité de craquage thermique à haute température ou dans lequel les hydrocarbures liquides autres que la fraction en huile synthétique utilisée dans le procédé selon l'invention sont aussi envoyés vers l'unité de craquage thermique à haute température.

12. Procédé selon l'une quelconque des revendications 1 à 11, dans lequel le méthane produit dans la réaction de Fischer Tropsch et tout hydrogène et monoxyde de carbone non converti sont utilisés comme carburant pour la réaction de craquage thermique doux et/ou le craquage thermique à haute température.

13. Procédé selon l'une quelconque des revendications 1 à 12, dans lequel des condensats de gaz naturel et/ou de l'éthane et/ou du propane extraits à partir de sources de gaz naturel sont aussi envoyés vers le procédé de craquage thermique à haute température.
